# DEMANDE DE BREVET EUROPEEN

(11) **EP 4 310 292 A1**
(43) Date de publication de la demande: **24.01.2024**
(21) Numéro de dépôt: 23184881.3
(22) Date de dépôt: 11.07.2023
(51) Int. Cl.: E06B 7/23, A61L 2/20, E06B 5/14, F16J 15/00

(54) **DISPOSITIF D'ÉTANCHÉITÉ ENTRE UN OUVRANT ET UN DORMANT D'UNE PORTE**

(30) Priorité: 19.07.2022 FR 2207398
(71) Demandeur: Guino3, 60220 Lannoy-Cuillère (FR)
(72) Inventeur: Noël, Jean-Pierre, 60220 Lannoy-Cuillère (FR)
(74) Mandataire: RVDB

(57) **Abrégé**

La présente invention concerne dispositif d'étanchéité (6) entre une paroi d'un ouvrant (2) et une paroi d'un dormant (3), lequel comprend deux joints semi-rigides (11, 12) espacés entre eux et un joint souple (13) placé entre les deux joints semi-rigides et associé à l'un (12) des deux joints semi-rigides. Dans un plan de coupe transversal, les joints semi-rigides présentent une forme de bloc ayant une première longueur et le joint souple présente une forme de languette ayant une seconde longueur, supérieure à la première longueur, et une partie d'extrémité (13b) débordant du joint semi-rigide auquel il est associé. La fermeture de l'ouvrant compresse l'au moins un joint semi-rigide entre la paroi de l'ouvrant et la paroi du dormant et incurve l'au moins un joint souple du côté de sa partie d'extrémité. Le dispositif d'étanchéité comprend un système de compression (15) configuré pour régler la pression à une première valeur Pl dans l'espace (14) entre le joint semi-rigide individuel (11) et le joint souple (13).

## Description

### Domaine technique

La présente invention concerne le domaine des dispositifs d'étanchéité mis en oeuvre entre un ouvrant et un dormant d'une porte séparant un premier environnement d'un second environnement. Un objectif recherché est d'assurer une parfaite étanchéité entre le premier environnement et le second environnement.

### Etat de la technique

De nombreuses applications nécessitent de préserver l'un de l'autre, deux environnements qui communiquent au moyen d'une porte, cette porte permettant également leur séparation physique de manière étanche. C'est par exemple le cas pour séparer une salle blanche, un environnement viral, bactérien ou chimique ou une zone exposée à des gaz ou des émanations toxiques, du milieu externe. C'est également le cas sur des installations permettant de traiter des produits placés dans une enceinte et introduits dans celle-ci grâce à la présence d'une porte d'accès, par exemple une installation de traitement à l'ozone.

Cette séparation physique entre les deux environnements nécessite la présence d'un dispositif d'étanchéité qui, dans de nombreux cas, se résume à un simple joint comprimé entre l'ouvrant et le dormant de la porte. Les risques de contamination du milieu ambiant par l'environnement à risque, dus par exemple à des virus, des bactéries ou des émanations toxiques ou, inversement, de l'environnement sain (par exemple, une salle blanche) à préserver du milieu ambiant, restent donc présents pour de nombreuses installations.

La Demanderesse a connaissance des publications des demandes de brevet WO2005118405A1 et US20150004061A1.

### Résumé de l'invention

La présente invention a pour objectif de concevoir un dispositif d'étanchéité entre une paroi d'un ouvrant et une paroi d'un dormant d'une porte qui assure une étanchéité optimisée afin de séparer physiquement de manière étanche un premier environnement et un second environnement au moyen de ladite porte, lorsque celle-ci est fermée. Le terme « porte » couvre tout dispositif d'ouverture/fermeture composé d'un ouvrant et d'un dormant, l'ouvrant étant mobile par rapport au dormant, par exemple par pivotement ou par coulissement, pour être déplacé dans une position d'ouverture permettant au premier environnement de communiquer avec le second environnement ou dans une position de fermeture pour séparer physiquement le premier environnement du second environnement. Il peut par exemple s'agir d'une porte en tant que telle, ou encore d'une trappe, d'une paroi vitrée coulissante, d'une porte à hublot, d'une plaque ou d'un couvercle amovible obstruant une ouverture ou un passage dans une position fermée et permettant aux deux environnements de communiquer dans une position ouverte.

L'invention concerne un dispositif d'étanchéité entre une paroi d'un ouvrant et une paroi d'un dormant qui comprend au moins deux joints semi-rigides présentant chacun dans un plan de coupe transversal une forme de bloc ayant une première longueur et au moins un joint souple présentant dans un plan de coupe transversale une forme de languette ayant une seconde longueur, la seconde longueur étant supérieure à la première longueur. On entend par « semi-rigide » un joint qui présente une dureté Shore comprise entre 50 et 100 afin de maintenir la forme du joint semi-rigide lorsqu'il est mis en compression. De même, on entend par « souple » un joint qui présente une dureté Shore comprise entre 25 et 50 de sorte assurer une déformation et le plaquage du joint souple contre la paroi de l'ouvrant ou du dormant lors d'une surpression dans la chambre.

Le premier environnement et le second environnement peuvent être à une même pression, notamment la pression atmosphérique, ou à des pressions différentes. Selon le dispositif d'étanchéité objet de l'invention, les deux j oints semi-rigides sont espacés entre eux et le joint souple est placé entre les deux j oints semi-rigides et associé à l'un des deux j oints semi-rigides. Les deux joints semi-rigides et le joint souple présentent les caractéristiques précitées. Ledit au moins un j oint souple présente une partie d'extrémité débordant dudit au moins un j oint semi-rigide auquel il est associé, de sorte que la fermeture de l'ouvrant compresse l'au moins un joint semi-rigide entre la paroi de l'ouvrant et la paroi du dormant et, concomitamment, incurve l'au moins un joint souple du côté de sa partie d'extrémité pour la plaquer contre la paroi de l'ouvrant ou la paroi du dormant, selon la configuration. En outre, le dispositif d'étanchéité comprend un système de compression configuré pour régler la pression à une première valeur P1 dans l'espace entre le joint semi-rigide individuel, c'est-à-dire celui qui n'est pas associé au joint souple, et ledit joint souple.

De préférence, le joint semi-rigide individuel est placé du côté de l'environnement à préserver de l'environnement à risque et le joint souple et le joint semi-rigide qui lui est associé sont placés du côté de l'environnement à risque, la valeur de la pression P1 sera supérieure à la pression dans l'environnement à préserver et aussi à la pression dans l'environnement à risque. A l'inverse, le joint semi-rigide individuel pourrait être placé du côté de l'environnement à risque et le joint souple et le joint semi-rigide qui lui est associé seraient alors placés du côté de l'environnement à préserver de l'environnement à risque, la valeur de la pression P1 étant toujours supérieure à la pression dans l'environnement à préserver et aussi à la pression dans l'environnement à risque.

L'homme du métier comprendra également que selon la situation, l'environnement à préserver et l'environnement à risque diffèrent. Selon un premier exemple, l'environnement à préserver est le milieu ambiant ou extérieur et l'environnement à risque est un milieu toxique ou infectieux. A l'inverse, selon un second exemple, l'environnement à risque est le milieu ambiant ou extérieur et l'environnement à préserver est une salle blanche.

L'invention ne se limite pas à la présence de deux joints semi-rigides et d'un joint souple, le dispositif d'étanchéité pouvant reproduire en cascade, une ou plusieurs fois, la présence d'un joint souple associé à un joint semi-rigide disposés les uns à la suite des autres avec des espacements, les pressions dans les espaces entre les joints semi-rigides et les joints souples étant dégressives en partant de l'environnement préservé, vers l'environnement à risque. Ainsi, selon une réalisation, le dispositif d'étanchéité selon l'invention comprend trois joints semi-rigides espacés successivement les uns des autres et deux joints souples placés respectivement entre les trois joints semi-rigides et associés à deux joints semi-rigides successifs, le système de compression étant configuré pour régler la pression à une première valeur P1 dans un premier espace entre le joint semi-rigide individuel, c'est-à-dire celui qui n'est pas associé au joint souple, et le premier joint souple associé à un premier joint semi-rigide et pour régler la pression à une seconde valeur P2 dans un second espace entre le premier joint semi-rigide et le second joint souple associé à un second joint semi-rigide, la première valeur de pression P1 étant supérieure à la seconde valeur de pression P2.

Selon une autre réalisation, le dispositif d'étanchéité selon l'invention comprend quatre joints semi-rigides espacés successivement les uns des autres et trois joints souples placés respectivement entre les quatre joints semi-rigides et associés à trois joints semi-rigides successifs, le système de compression étant configuré pour régler la pression à une première valeur P1 dans un premier espace entre le joint semi-rigide individuel, c'est-à-dire celui qui n'est pas associé au joint souple, et le premier joint souple associé à un premier joint semi-rigide, pour régler la pression à une seconde valeur P2 dans un second espace entre le premier joint semi-rigide et le second joint souple associé à un second joint semi-rigide et pour régler la pression à une troisième valeur P3 dans un troisième espace entre le second joint semi-rigide et le troisième joint souple associé à un troisième joint semi-rigide, la première valeur de pression P1 étant supérieure à la seconde valeur de pression P2 et la seconde valeur de pression P2 étant supérieure à la troisième valeur de pression P3.

Selon une réalisation préférentielle du dispositif d'étanchéité, la première valeur de pression P1 est de huit cent vingt millimètres de mercure (820 mm Hg, soit 1093 hPa), la seconde valeur de pression P2 est de huit cents millimètres de mercure (800 mm Hg, soit 1067 hPa) et la troisième valeur de pression P3 est de sept cent quatre-vingts millimètres de mercure (780 mm Hg, soit 1040 hPa). Cette mise en oeuvre est particulièrement adaptée pour un premier environnement et un second environnement à la pression atmosphérique, c'est-à-dire une pression de 760 millimètres de mercure (760 mm Hg, soit 1013 hPa).

Selon une réalisation préférentielle, la différence de pression entre la première valeur de pression P1 et la seconde valeur de pression P2 est de vingt millimètre de mercure (20 mm Hg, soit 2666 Pa). De la même manière, la différence de pression entre la seconde valeur de pression P2 et la troisième valeur de pression P3 est de vingt millimètres de mercure (20 mm Hg, soit 2666,45 Pa).

Selon une réalisation du dispositif d'étanchéité objet de l'invention, le système de compression comprend un organe de contrôle de la pression entre deux joints semi-rigides successifs, de préférence un pressostat. Ainsi, selon la conception du dispositif d'étanchéité, ledit organe de contrôle permet de contrôler la première valeur de pression P1 (en la présence d'un, deux ou trois espaces), la seconde valeur de pression P2 (en la présence de deux ou trois espaces) et la troisième valeur de pression P3 (en la présence de trois espaces). De préférence, le dispositif d'étanchéité selon l'invention comprend en complément un système d'alarme configuré pour signaler une anomalie de pression entre deux joints semi-rigides successifs.

L'invention concerne également une installation comprenant un ouvrant et un dormant séparant un premier environnement d'un second environnement, ladite installation comprenant un dispositif d'étanchéité agencé entre une paroi de l'ouvrant et une paroi du dormant, lesdites parois étant aptes à coopérer lors de la fermeture de l'ouvrant par l'intermédiaire dudit dispositif d'étanchéité qui comporte les caractéristiques de l'une quelconque des caractéristiques précitées. Il peut s'agir, par exemple, d'une installation comprenant une salle blanche ou un environnement toxique ou infectieux, séparé de l'environnement externe par une porte de sas, ou encore une machine effectuant un traitement sur des produits dans une enceinte, au moyen d'un liquide ou d'un gaz nocif, l'enceinte étant séparé de l'environnement externe par une porte.

Selon une réalisation de l'installation objet de l'invention, celle-ci est une installation de décontamination d'articles à l'ozone qui comprend un caisson étanche définissant une enceinte et comprenant une porte d'ouverture et de fermeture étanche, laquelle permet d'accéder à l'enceinte. L'installation de décontamination comprend également un système de production d'ozone dans l'enceinte, un système de traitement de l'ozone, le dispositif d'étanchéité étant mis en oeuvre entre l'ouvrant de la porte et le dormant de la porte agencée sur le caisson.

### Brève description des figures

Les caractéristiques et avantages de l'invention apparaîtront à la lecture de la description suivante s'appuyant sur des figures, parmi lesquelles :
[Fig. 1] La figure 1 schématise une première réalisation du dispositif d'étanchéité, mise en oeuvre entre un ouvrant et un dormant ;
[Fig. 2] La figure 2 schématise une seconde réalisation du dispositif d' étanchéité, mise en oeuvre entre un ouvrant et un dormant ;
[Fig. 3] La figure 3 schématise une troisième réalisation du dispositif d'étanchéité, mise en oeuvre entre un ouvrant et un dormant, selon une première conception de la porte ;
[Fig. 4] La figure 4 schématise la troisième réalisation du dispositif d'étanchéité illustré figure 3, mise en oeuvre entre un ouvrant et un dormant, selon une seconde conception de la porte ;
[Fig.5] La figure 5 schématise une installation de traitement à l'ozone pour la désinfection de produits.

### Description détaillée

Dans la suite de la description, les mêmes références sont utilisées pour désigner les mêmes caractéristiques selon les diverses variantes de réalisation, sans indication contraire dans le texte.

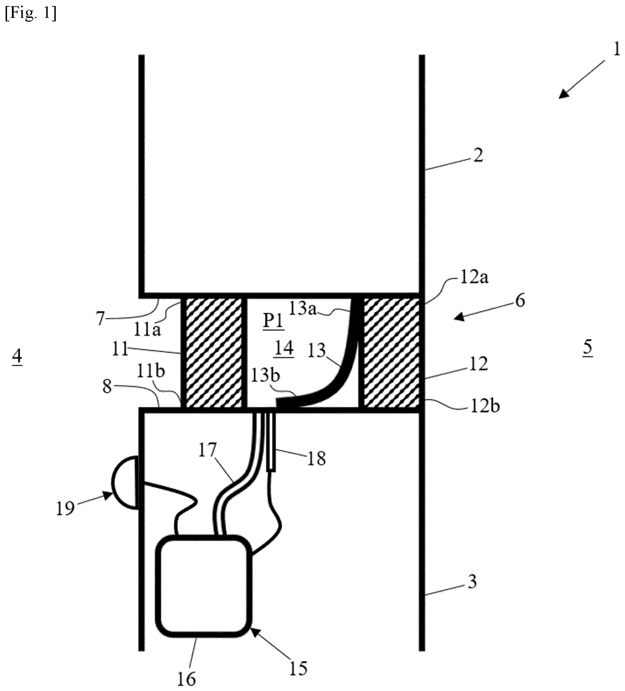

Sur la figure 1, est illustrée partiellement une porte 1 qui comprend un ouvrant 2 et un dormant 3, la porte 1 permettant une séparation physique entre un premier environnement 4 et un second environnement 5. Le premier environnement 4 est par exemple le milieu ambiant et le second environnement 5 est par exemple une salle ou une enceinte toxique ou infectieuse. Dans la position fermée telle qu'illustrée sur la figure 1, un dispositif d'étanchéité 6 assure une étanchéité entre le chant 7 de l'ouvrant 2 et le chant 8 du dormant 3. L'ouverture et la fermeture de la porte 1 peuvent se faire par exemple en pivotement l'ouvrant 2 par rapport au dormant 3 au moyen de charnières (non illustrées), ou en coulissant l'ouvrant 2 par rapport au dormant 3 au moyen d'une glissière. Le dispositif d'étanchéité 6 est agencé entre le chant 7 de l'ouvrant 2 et le chant 8 du dormant 3.

Sur la figure 1, le dispositif d'étanchéité 6 comprend un joint semi-rigide individuel 11, auquel il n'est associé aucun joint souple, et un premier joint semi-rigide 12 auquel est associé un premier joint souple 13. Le joint semi-rigide individuel 11 est identique au premier joint semi-rigide 12, excepté la présence du premier joint souple 13 associé audit premier joint semi-rigide 12. Sur la figure 1, le joint semi-rigide individuel 11, le premier joint semi-rigide 12 et le premier joint souple 13 sont assujettis au chant 7 de l'ouvrant 2, par exemple par collage ou par un emboîtement en force des premières parties d'extrémité 11a, 12a, 13a respectivement du joint semi-rigide individuel 11, du premier joint semi-rigide 12 et du premier joint souple 13, dans le chant 7 de l'ouvrant 2. La première partie d'extrémité 12a du premier joint semi-rigide 12 et la première partie d'extrémité 13a du premier joint souple 13 peuvent être assemblées entre elles pour réaliser ladite association, par exemple par collage ou soudage ; cette association peut aussi être obtenue du fait dudit assujettissement des premières parties d'extrémité 12a, 13a, juxtaposées, avec le chant 7 de l'ouvrant 2.

Sur la figure 1, le joint semi-rigide individuel 11 et le premier joint semi-rigide 12 présentent dans un plan de coupe transversal illustré sur ladite figure 1, une forme de bloc qui est de préférence rectangulaire, une forme carrée ou ovale étant toutefois possible. Le premier joint souple 13 présente dans ce plan de coupe transversal une forme de languette dont la longueur est supérieure à celle du premier joint semi-rigide 12 de sorte que la seconde partie d'extrémité 13b du premier joint souple 10 dépasse largement la seconde partie d'extrémité 12b du premier joint semi-rigide 12 dans une position relâchée desdits j oints où la porte 1 est en position ouverte (non illustrée). Ainsi, lorsque l'ouvrant 2 ferme le dormant 3, les secondes parties d'extrémité 11b, 12b du joint semi-rigide individuel 11 et du premier joint semi-rigide 12 appuient contre le chant 8 du dormant 3, ce qui comprime le joint semi-rigide individuel 11 et le premier joint semi-rigide 12, tandis que la seconde partie d'extrémité 13b du premier joint souple 10 se déforme pour donner une forme incurvée audit premier joint souple 13, comme le montre la figure 1. La mise en oeuvre pourrait être inversée, le joint semi-rigide individuel 11, le premier joint semi-rigide 12 et le premier joint souple 13 pouvant être assujettis au chant 8 du dormant 3 et prendre appui contre le chant 7 de l'ouvrant 2, lors de la fermeture de la porte 1. Le joint semi-rigide individuel 11 est distant du premier joint semi-rigide 12, laissant ainsi un premier espace 14 entre le joint semi-rigide individuel 11 et le premier joint souple 13 associé au premier joint semi-rigide 12.

Selon cette réalisation de la figure 1, le dispositif d'étanchéité 6 comprend un système de compression 15 comprenant premier compresseur 16 et un premier circuit 17 qui débouche dans le premier espace 14, lorsque l'ouvrant 2 est fermé. Sur la figure 1, le système de compression 15 est agencé sur le dormant 3 et le premier circuit 17 débouche sur le chant 8 dudit dormant 3. Dans une variante, le système de compression 15 pourrait être agencé sur l'ouvrant 2, dans quel cas le premier circuit 17 déboucherait sur le chant 7 dudit ouvrant 2. Lorsque la porte 1 est fermée, le joint semi-rigide individuel 11 et le premier joint semi-rigide 12 sont comprimés entre le chant 7 de l'ouvrant 2 et le chant 8 du dormant 3 et le premier joint souple 13 se déforme et prend une forme incurvée, comme le montre la figure 1. Le système de compression 15 permet d'augmenter la valeur de la pression P1 dans le premier espace 14, ce qui permet de plaquer le premier joint souple 13 contre le chant 8 du dormant 3 et contre le premier joint semi-rigide 12, ladite valeur de pression P1 étant supérieure aux valeurs de pression dans le premier environnement 4 et dans le second environnement 5. Par exemple, lorsque le premier environnement 4 et le second environnement 5 sont à la pression atmosphérique, soit 760 millimètres de mercure (760 mm Hg, soit 1013 hPa), la valeur de pression P1 sera de préférence réglée à 780 millimètres de mercure (780 mm Hg, soit 1040 hPa).

Le système de compression 15 peut comprendre, en complément, un premier organe de contrôle 18 de la pression entre le joint semi-rigide individuel 11 et le premier joint souple 13, de préférence un pressostat, schématisé figure 1, qui relève la valeur de pression P1 dans le premier espace 14. En cas de variation anormale de la valeur de pression P1 en dessous d'une valeur de référence, par exemple fixée à 770 millimètres de mercure (770 mm Hg, soit 1027 hPa), le système de compression 15 déclenche un système d'alarme 19 configuré pour signaler l'anomalie de pression. Ce système d'alarme 19 peut par exemple consister en une sirène, en un voyant lumineux, un écran de signalisation ou en une combinaison de ces systèmes.

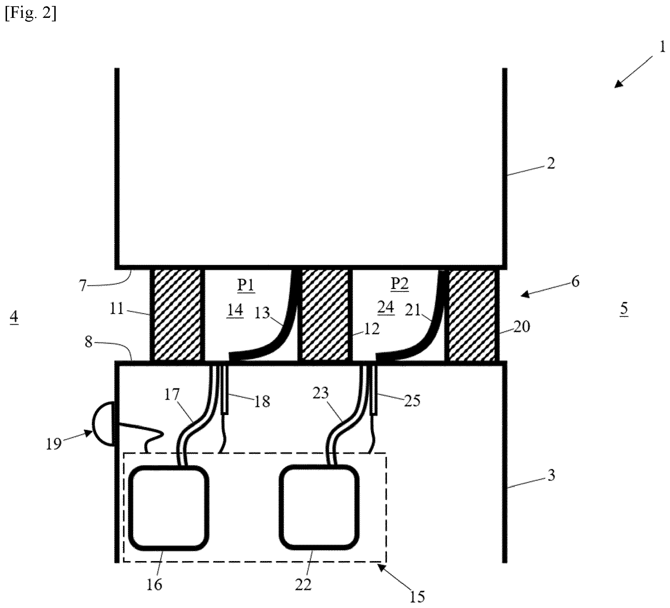

La figure 2 reprend les caractéristiques décrites précédemment pour la figure 1 et y ajoute un second joint semi-rigide 20 associé à un second joint souple 21, avec un espacement par rapport au premier joint semi-rigide 12. Le second joint semi-rigide 20 et le second joint souple 21 sont identiques respectivement au premier joint semi-rigide 12 et au premier joint souple 13. Le système de compression 15 comprend en complément un second compresseur 22 et un second circuit 23 qui débouche dans un second espace 24 lorsque l'ouvrant 2 est fermé, ledit second espace 24 étant situé entre le premier joint semi-rigide 12 et le second joint souple 21 associé au second joint semi-rigide 20. Sur la figure 2, le système de compression 15 est agencé sur le dormant 3 et le premier circuit 17 et le second circuit 23 débouchent sur le chant 8 dudit dormant 3. Dans une variante, le système de compression 15 pourrait être agencé sur l'ouvrant 2, dans quel cas le premier circuit 17 et le second circuit 23 déboucheraient sur le chant 7 dudit ouvrant 2.

Sur la figure 2, lorsque la porte 1 est fermée, le joint semi-rigide individuel 11, le premier joint semi-rigide 12 et le second joint semi-rigide 20 sont comprimés entre le chant 7 de l'ouvrant 2 et le chant 8 du dormant 3 et le premier j oint souple 13 et le second j oint souple 21 se déforment et prennent une forme incurvée, comme le montre la figure 2. Le système de compression 15 permet d'augmenter la valeur de la pression P1 dans le premier espace 14 et la valeur de pression P2 dans le second espace 24, en conservant une valeur de pression P1 supérieure à la valeur de pression P2, ce qui permet de plaquer le premier joint souple 13 contre le chant 8 du dormant 3 et contre le premier joint semi-rigide 12 et le second joint souple 21 contre le chant 8 du dormant 3 et contre le second joint semi-rigide 20, lesdites valeurs de pression P1 et P2 étant supérieures aux valeurs de pression dans le premier environnement 4 et dans le second environnement 5. Par exemple, lorsque le premier environnement 4 et le second environnement 5 sont à la pression atmosphérique, soit 760 millimètres de mercure (760 mm Hg, soit 1013 hPa), la valeur de pression P1 sera de préférence réglée à 800 millimètres de mercure (800 mm Hg, soit 1067 hPa) et la valeur de pression P2 sera de préférence réglée à 780 millimètres de mercure (780 mm Hg, soit 1040 hPa).

Le système de compression 15 peut comprendre, en complément :
- un premier organe de contrôle 18 de la pression entre le joint semi-rigide individuel 11 et le premier joint souple 13, de préférence un pressostat, schématisé figure 2, qui relève la valeur de pression P1 dans le premier espace 14 ;
- un second organe de contrôle 25 de la pression entre le premier joint semi-rigide 12 et le second joint souple 21, de préférence un pressostat, schématisé figure 2, qui relève la valeur de pression P2 dans le second espace 24.

En cas de variation anormale de la valeur de pression P1 et/ou de la valeur de pression P2, en dessous de valeurs de référence, par exemple fixée à 790 millimètres de mercure (790 mm Hg, soit 1053 hPa) pour la première valeur de pression P1 et à 770 millimètres de mercure (770 mm Hg, soit 1027 hPa) pour la seconde valeur de pression P2, le système de compression 15 déclenche un système d'alarme 19 configuré pour signaler l'anomalie de pression. Ce système d'alarme 19 peut par exemple consister en une sirène, en un voyant lumineux, un écran de signalisation ou en une combinaison de ces systèmes.

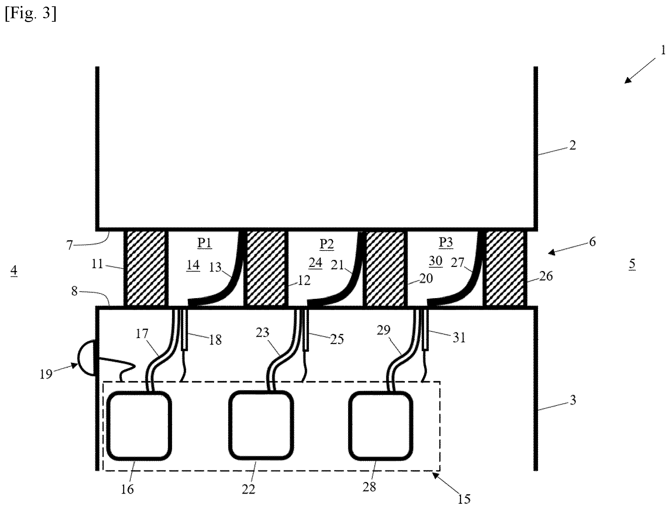

La figure 3 reprend les caractéristiques décrites précédemment pour les figures 1 et 2 et y ajoute un troisième joint semi-rigide 26 associé à un troisième joint souple 27, avec un espacement par rapport au second joint semi-rigide 20. Le troisième joint semi-rigide 26 et le troisième joint souple 27 sont identiques respectivement aux premier et second joints semi-rigides 12, 20 et aux premier et second joints souples 13, 21. Le système de compression 15 comprend en complément un troisième compresseur 28 et un troisième circuit 29 qui débouche dans un troisième espace 30 lorsque l'ouvrant 2 est fermé, ledit troisième espace 30 étant situé entre le second joint semi-rigide 20 et le troisième j oint souple 27 associé au troisième joint semi-rigide 26. Sur la figure 3, le système de compression 15 est agencé sur le dormant 3 et le premier circuit 17, le second circuit 23 et le troisième circuit 29 débouchent sur le chant 8 dudit dormant 3. Dans une variante, le système de compression 15 pourrait être agencé sur l'ouvrant 2, dans quel cas le premier circuit 17, le second circuit 23 et le troisième circuit 29 déboucheraient sur le chant 7 dudit ouvrant 2.

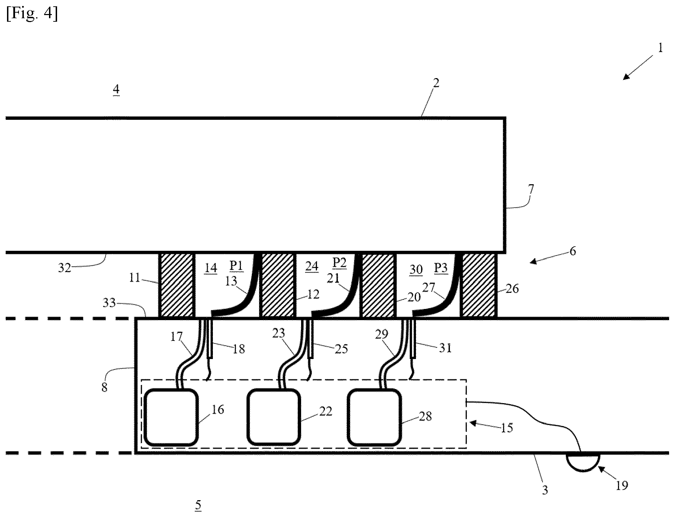

Sur la figure 3, lorsque la porte 1 est fermée, le joint semi-rigide individuel 11, le premier joint semi-rigide 12, le second joint semi-rigide 20 et le troisième joint semi-rigide 26 sont comprimés entre le chant 7 de l'ouvrant 2 et le chant 8 du dormant 3 et le premier joint souple 13, le second joint souple 21 et le troisième joint souple 27 se déforment et prennent une forme incurvée, comme le montre la figure 4. Le système de compression 15 permet d'augmenter la valeur de la pression P1 dans le premier espace 14, la valeur de pression P2 dans le second espace 24 et la valeur de la pression P3 dans le troisième espace 30, en conservant une valeur de pression P1 supérieure à la valeur de pression P2, elle-même supérieure à la valeur de pression P3, ce qui permet de plaquer le premier joint souple 13 contre le chant 8 du dormant 3 et contre le premier joint semi-rigide 12, le second joint souple 21 contre le chant 8 du dormant 3 et contre le second joint semi-rigide 20 et le troisième joint souple 27 contre le chant 8 du dormant et contre le troisième joint semi-rigide 26, lesdites valeurs de pression P1, P2 et P3 étant supérieures aux valeurs de pression dans le premier environnement 4 et dans le second environnement 5. Par exemple, lorsque le premier environnement 4 et le second environnement 5 sont à la pression atmosphérique, soit 760 millimètres de mercure (760 mm Hg, soit 1013 hPa), la valeur de pression P1 sera de préférence réglée à 820 millimètres de mercure (820 mm Hg, soit 1093 hPa), la valeur de pression P2 sera de préférence réglée à 800 millimètres de mercure (800 mm Hg, soit 1067 hPa) et la valeur de pression P3 sera de préférence réglée à 780 millimètres de mercure (780 mm Hg, soit 1040 hPa).

Le système de compression 15 peut comprendre, en complément :
- un premier organe de contrôle 18 de la pression entre le joint semi-rigide individuel 11 et le premier joint souple 13, de préférence un pressostat, schématisé figure 3, qui relève la valeur de pression P1 dans le premier espace 14 ;
- un second organe de contrôle 25 de la pression entre le premier joint semi-rigide 12 et le second joint souple 21, de préférence un pressostat, schématisé figure 3, qui relève la valeur de pression P2 dans le second espace 24 ;
- un troisième organe de contrôle 31 de la pression entre le second joint semi-rigide 20 et le troisième joint souple 27, de préférence un pressostat, schématisé figure 3, qui relève la valeur de pression P3 dans le troisième espace 30.

En cas de variation anormale de la valeur de pression P1, de la valeur de pression P2 et/ou de la valeur de pression P3, en dessous de valeurs de référence, par exemple fixées à 810 millimètres de mercure (810 mm Hg, soit 1080 hPa) pour la première valeur de pression P1, à 790 millimètres de mercure (790 mm Hg, soit 1053 hPa) pour la seconde valeur de pression P2 et à 770 millimètres de mercure (770 mm Hg, soit 1027 hPa) pour la troisième valeur de pression P3, le système de compression 15 déclenche un système d'alarme 19 configuré pour signaler l'anomalie de pression. Ce système d'alarme 19 peut par exemple consister en une sirène, en un voyant lumineux, un écran de signalisation ou en une combinaison de ces systèmes.

La figure 4 montre une variante qui reprend en tout point les caractéristiques de la figure 3 décrites ci-avant, excepté le fait que le dispositif d'étanchéité 6 n'est pas agencé entre le chant 7 de l'ouvrant 2 et le chant 8 du dormant 3, mais entre une face 32 de l'ouvrant 2 et une face 33 du dormant 3, la porte 1 étant une porte coulissante.

Les joints semi-rigides 11, 12, 20, 26 présentent une dureté Shore sur une échelle de 0 à 100, comprise entre 50 et 100 et les joints souples 13, 21, 27 présentent une dureté Shore sur une échelle de 0 à 100, comprise entre 25 et 50 leur permettant de subir des déformations de 30 à 50% et, ainsi, d'assurer une étanchéité de par sa souplesse et les surpressions créées dans les espaces 14, 24, 30 entre lesdits joints semi-rigides 11, 12, 20 et les joints souples 13, 21, 27. De préférence, la matière choisie pour tous ces j oints semi-rigides 11, 12, 20, 26 et souples 13, 21, 27 est de l'éthylène propylène diène-monomère (EPDM).

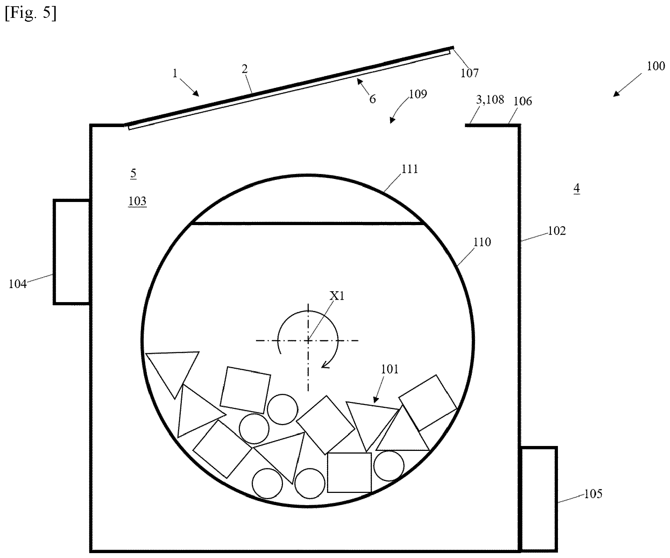

Le dispositif d'étanchéité 6 peut être mis en oeuvre sur tout type d'installation nécessitant de séparer physiquement et de manière étanche un premier environnement 4 d'un second environnement 5, au moyen d'une porte 1. Dans une mise en oeuvre schématisée en figure 5, il s'agit d'une installation 100 de décontamination d'articles 101 à l'ozone qui comprend un caisson 102 étanche, définissant une enceinte 103 et comprenant une porte 1 d'ouverture et de fermeture étanche permettant d'accéder à l'enceinte 103.

Sur la figure 5, l'installation 100 comprend également un système de production d'ozone 104 dans l'enceinte 103 et un système de traitement de l'ozone 105. Le système de production d'ozone 104 est constitué d'un ozoneur (non schématisé) qui transforme l'oxygène en oxygène triatomique et permet d'obtenir un mélange d'air et d'ozone dont les pourcentages seront définis suivant un barème volume, temps et concentration adaptés à l'enceinte 103 du caisson 102. L'ozone a un pouvoir bactéricide et virucide très élevé agissant par oxydation ; il est ainsi en mesure de détruire les virus quelle que soit leur mutation. Le système de traitement de l'ozone 105 comprend un aspirateur (non schématisé), de préférence positionné en partie basse de l'enceinte 103, qui extrait l'ozone de l'enceinte 103 et un catalyseur thermique (non schématisé) qui transforme l'oxygène triatomique en oxygène. L'ozone est avantageusement très écologique car il utilise l'oxygène pour se transformer en oxygène triatomique et se retransformer en oxygène après passage dans le catalyseur thermique. En fin de cycle de décontamination, l'air ozoné présent dans l'enceinte 103 est aspiré et envoyé à travers le catalyseur thermique qui retransforme l'ozone non consommé en oxygène afin de respecter les normes de rejet.

Sur la figure 5, le dispositif d'étanchéité 6 est mis en oeuvre entre l'ouvrant 2 de la porte 1 et le dormant 3 de la porte 1, ledit dormant 3 étant formé sur la paroi supérieure 106 du caisson 102. Le dispositif d'étanchéité 6 sera agencé sur le bord périphérique 107 de la face interne 2a de l'ouvrant 2 et prendra appui contre le contour 108 formant l'ouverture 109 sur la paroi supérieure 106.

L'installation 100 comprend également une cage 110 présentant, sur la figure 5, la forme d'un tambour grillagé muni d'une trappe d'accès 111 pour l'introduction et le retrait des articles 101 à traiter à l'ozone, ladite cage 110 étant activée en rotation selon un axe X1 pour agiter les articles 101 et traiter toutes leurs surfaces à l'ozone. Bien entendu, l'installation 100 pourrait prendre d'autres formes tout en assurant une décontamination des articles 101 à l'ozone et en comportant un dispositif d'étanchéité 6 entre l'ouvrant 2 et le dormant 3 de la porte 1 d'accès à l'enceinte 103 du caisson 102.

Des variantes sont envisageables dans le cadre de l'invention, par exemple quant à l'association du joint semi-rigide 12, 20, 26 et du joint souple 13, 21, 27. Dans les exemples qui précèdent, le joint semi-rigide et le joint souple sont deux pièces distinctes associées par assemblage entre elles (collage ou soudage, par exemple) et/ou avec la paroi du dormant 2, voire la paroi de l'ouvrant 3, selon l'agencement prévu. Le joint semi-rigide 12, 20, 26 et le joint souple 13, 21, 27 peuvent être constitués d'une seule pièce moulée bi-matière, la première matière mettant en oeuvre le joint semi-rigide et la seconde matière mettant en oeuvre le joint souple, voire monomatière, dans quel cas la souplesse et la semi-rigidité dépendront de l'épaisseur de la forme en bloc et de l'épaisseur de la forme en languette.

## Revendications

1. Dispositif d'étanchéité (6) entre une paroi d'un ouvrant (2) et une paroi d'un dormant (3), **caractérisé en ce qu'**il comprend deux joints semi-rigides (11, 12) espacés entre eux et un joint souple (13) placé entre les deux joints semi-rigides et associé à l'un (12) des deux joints semi-rigides, les joints semi-rigides (11, 12) présentant dans un plan de coupe transversal une forme de bloc ayant une première longueur et le joint souple (13) présentant dans un plan de coupe transversale une forme de languette ayant une seconde longueur, supérieure à la première longueur, ledit joint souple présentant une partie d'extrémité (13b) débordant du joint semi-rigide (12) auquel il est associé, de sorte que la fermeture de l'ouvrant compresse les joints semi-rigides (11, 12) entre la paroi de l'ouvrant et la paroi du dormant et incurve le joint souple du côté de sa partie d'extrémité, ledit dispositif d'étanchéité comprenant un système de compression (15) configuré pour régler la pression à une première valeur P1 dans l'espace (14) entre le joint semi-rigide individuel (11) et le joint souple (13).

2. Dispositif d'étanchéité (6) selon la revendication 1, lequel comprend trois joints semi-rigides (11, 12, 20) espacés successivement les uns des autres et deux joints souples (13, 21) placés respectivement entre les trois joints semi-rigides et associés à deux joints semi-rigides successifs (12, 20), le système de compression (15) étant configuré pour régler la pression à une première valeur P1 dans un premier espace (14) entre le joint semi-rigide individuel (11) et le premier joint souple (13) associé à un premier joint semi-rigide (12) et pour régler la pression à une seconde valeur P2 dans un second espace (24) entre le premier joint semi-rigide (12) et le second joint souple (21) associé à un second joint semi-rigide (20), P1 étant supérieure à P2.

3. Dispositif d'étanchéité (6) selon la revendication 2, lequel comprend quatre joints semi-rigides (11, 12, 20, 26) espacés successivement les uns des autres et trois joints souples (13, 21, 27) placés respectivement entre les quatre joints semi-rigides et associés à trois joints semi-rigides successifs (12, 20, 26), le système de compression (15) étant configuré pour régler la pression à une première valeur P1 dans un premier espace (14) entre le joint semi-rigide individuel (11) et le premier joint souple (13) associé à un premier joint semi-rigide (12), pour régler la pression à une seconde valeur P2 dans un second espace (24) entre le premier joint semi-rigide (12) et le second joint souple (21) associé à un second joint semi-rigide (20) et pour régler la pression à une troisième valeur P3 dans un troisième espace (30) entre le second joint semi-rigide (20) et le troisième joint souple (27) associé à un troisième joint semi-rigide (26), P1 étant supérieure à P2 et P2 étant supérieure à P3.

4. Dispositif d'étanchéité (6) selon l'une quelconque des revendications 2 ou 3, dans lequel la différence de pression entre la première valeur P1 et la seconde valeur P2 est de vingt millimètre de mercure (20 mm Hg).

5. Dispositif d' étanchéité (6) selon la revendication 4 dépendant de la revendication 3, dans lequel la différence de pression entre la seconde valeur P2 et la troisième valeur P3 est de vingt millimètres de mercure (20 mm Hg)

6. Dispositif d'étanchéité (6) selon la revendication 5, dans lequel la première valeur de pression P1 est de huit cent vingt millimètres de mercure (820 mm Hg), la seconde valeur de pression P2 est de huit cents millimètres de mercure (800 mm Hg) et la troisième valeur de pression P3 est de sept cent quatre-vingts millimètres de mercure (780 mm Hg).

7. Dispositif d'étanchéité (6) selon l'une quelconque des revendications 1 à 6, dans lequel le système de compression (15) comprend un organe de contrôle (18, 25, 31) de la pression entre deux joints semi-rigides successifs (11, 12, 20, 26), de préférence un pressostat.

8. Dispositif d'étanchéité (6) selon la revendication 7, lequel comprend un système d'alarme (19) configuré pour signaler une anomalie de pression entre deux joints semi-rigides successifs (11, 12, 20, 26).

9. Installation comprenant un ouvrant (2) et un dormant (3) séparant un premier environnement (4) d'un second environnement (5), ladite installation comprenant un dispositif d'étanchéité (6) qui comporte les caractéristiques de l'une quelconque des revendications 1 à 8, le dispositif d'étanchéité (6) étant agencé entre une paroi de l'ouvrant et une paroi du dormant, lesdites parois étant apte à coopérer lors de la fermeture de l'ouvrant par l'intermédiaire dudit dispositif d'étanchéité (6).

10. Installation selon la revendication 9, laquelle est une installation (100) de décontamination d'articles (101) à l'ozone qui comprend un caisson (102) étanche qui définit une enceinte (103) et comprend une porte (1) d'ouverture et de fermeture étanche permettant d'accéder à l'enceinte, un système de production d'ozone (104) dans l'enceinte, un système de traitement de l'ozone (105), le dispositif d'étanchéité (6) étant mis en oeuvre entre l'ouvrant (2) de la porte et le dormant (3) de la porte agencée sur le caisson.
